(19) Europäisches Patentamt / European Patent Office / Office européen des brevets

(11) **EP 4 250 193 A1**

(12) **EUROPEAN PATENT APPLICATION**

(43) Date of publication:
27.09.2023 Bulletin 2023/39

(21) Application number: 22167776.8

(22) Date of filing: 12.04.2022

(51) International Patent Classification (IPC):
*G06Q 10/00* (2023.01)    *G16H 40/40* (2018.01)
*G06F 1/16* (2006.01)    *G06F 3/01* (2006.01)
*G06F 3/03* (2006.01)

(52) Cooperative Patent Classification (CPC):
G06F 1/163; G06F 1/1686; G06F 3/011;
G06F 3/012; G06F 3/013; G06F 3/0304;
G06Q 10/20; G16H 40/40

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 25.03.2022 US 202263323659 P

(71) Applicant: Koninklijke Philips N.V.
5656 AG Eindhoven (NL)

(72) Inventors:
• BASTIANELLI, Emanuele
Eindhoven (NL)
• STOLIKJ, Milosh
Eindhoven (NL)
• GAO, Qi
Eindhoven (NL)
• BARBIERI, Mauro
Eindhoven (NL)

(74) Representative: Philips Intellectual Property &
Standards
High Tech Campus 52
5656 AG Eindhoven (NL)

(54) **GHOST MODE GLASSES FOR USE IN SERVICING MEDICAL DEVICES AND SERVICING WORKFLOWS USING SAME**

(57)    A non-transitory computer readable medium **(30)** stores data including recorded historical service call videos **(32)** for one or more medical imaging devices **(12),** the videos being annotated with information about the service calls; and instructions readable and executable by at least one electronic processor **(20)** to: control a camera **(14)** to acquire video **(34)** of a current medical device being serviced in a current service call; select one of the historical service call videos based on the acquired video; and provide, on a display device **(24),** the selected historical service call video.

Fig. 1

EP 4 250 193 A1

## Description

FIELD OF THE INVENTION

The following relates generally to the medical device arts, medical device maintenance arts, augmented reality (AR) arts, and related arts.

BACKGROUND OF THE INENTION

Medical devices occasionally exhibit a malfunction, which requires maintenance. Depending on the malfunction, the problem can sometimes be handled remotely, by a remote service engineer (RSE). The RSE can perform remote diagnostic by analyzing the log data generated from the malfunctioning medical device, or by remotely executing diagnostic tests. In certain situations, the RSE can successfully resolve the issue completely remotely.

However, for certain malfunctions, remote resolution is not possible. In such situations, in addition to the remote diagnostic, a field service engineer (FSE) visits the physical location of the medical device and perform on-site maintenance. The FSE typically has a report of the main findings from the RSE, and based on this information, they may need to perform additional troubleshooting steps to discover the root cause of the issue, and/or replace parts of the machine and/or perform other types of maintenance or repair.

While performing field service of medical devices, an FSE executes checking and repair actions on the device, e.g., running diagnostic tests, checking equipment for physical damage, replacing parts etc. Typically, the FSE relies on comparing the medical device under service with textual description and/or drawings of the actions to be performed that are provided in maintenance manuals (paper or electronic, e.g. in pdf format). This can be challenging since the textual descriptions require translation by the FSE to the observed physical device, and even drawings may not be sufficient since they typically show a single viewpoint (e.g. in a perspective view) or again require translation by the FSE to the observed physical device (e.g., in front/top/side drawing views). The following discloses certain improvements to overcome these problems and others.

SUMMARY OF THE INVETION

In one aspect, a non-transitory computer readable medium stores data including recorded historical service call videos for one or more medical imaging devices, the videos being annotated with information about the service calls; and instructions readable and executable by at least one electronic processor to: control a camera to acquire video of a current medical device being serviced in a current service call; select one of the historical service call videos based on the acquired video; and provide, on a display device, the selected historical service call video.

In another aspect, a system for providing maintenance videos for one or more medical devices includes an augmented-reality heads-up display (AR-HUD) device including a camera and at least one display device. A server computer is configured to: control the camera to acquire video of a current medical device being serviced in a current service call; select one or more historical service call videos based on the acquired video, the historical service call videos comprising pre-recorded historical service call videos for one or more medical imaging devices, the videos being annotated with information about the service calls; and provide, on the display device, the one or more selected historical service call video as a transparent or semi-transparent overlay.

In another aspect, a system for providing maintenance videos for one or more medical devices includes an AR-HUD device with at least one display device. A server computer is configured to: select one or more historical service call videos, the historical service call videos comprising pre-recorded historical service call videos for one or more medical imaging devices, the videos being annotated with information about the service calls; and provide, on the display device, the one or more selected historical service call video as a transparent or semi-transparent overlay.

One advantage resides in using pre-recorded videos of repair actions presented on an augmented reality (AR) device to assist an engineer in performing maintenance on a medical device. Another advantage resides in providing an efficient methodology for creating AR content for such AR-assisted maintenance.

Another advantage resides in displaying pre-recorded videos of repair actions on a device to overlap with the engineer's field of view, guiding the engineer's action, or suggesting possible actions. Another advantage resides in using pre-recorded videos of repair actions on a device in lieu of maintaining AR technical content.

A given embodiment may provide none, one, two, more, or all of the foregoing advantages, and/or may provide other advantages as will become apparent to one of ordinary skill in the art upon reading and understanding the present disclosure.

BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

Fig. 1 diagrammatically illustrates an illustrative system for selection videos for display in accordance with the present disclosure.
Fig. 2 shows exemplary flow chart operations of the system of Fig. 1.

DETAILED DESCRIPTION OF EMBODIMENTS

In embodiments disclosed herein, servicing of medical devices benefit from a support capable of displaying the actions to be executed, in way that the displayed information overlaps with the engineer's field of view, thereby guiding the engineer's action, or suggesting possible actions. The disclosed embodiments are implemented through Augmented Reality (AR), with relevant digital content being added as AR content in real-time on overlaid on the FSE point-of-view, by using AR glasses, goggles, or the like.

More particularly, the following discloses use of AR glasses or an AR headset to provide a field service engineer (FSE) with visual assistance in repairing a medical imaging device. A database of recorded historical service call videos, annotated with information about the service calls, is maintained. Each video is acquired by a FSE, for example using a forward-looking camera of AR glasses or an AR headset worn by the FSE performing the historical service call. (In a variant embodiment, the historical video may be acquired using a forward-looking camera mounted on the head of the FSE, but without the camera mount providing AR presentation capability). Thus, a large database of historical service call videos can be created, with minimal archival effort limited to selecting the "best" service call videos for inclusion in the database (e.g., a failed service call in which the FSE failed to resolve the problem would preferably be excluded from the database) and adding informative metadata such as the make/model of medical imaging device and the type of service shown in the video. A video retrieval component utilizes the metadata to select a most similar video which is then presented to the FSE handling the current service call. In the illustrative examples, the service call video is presented by the AR glasses or headset as a transparent or semi-transparent overlay (also referred to herein as a "ghost" display due to its transparent or semi-transparent nature). Since the video will show the (historical) imaging device from the vantage of the historical FSE who recorded it, this may not match up with the vantage the current FSE is viewing the (current) imaging device, but this can be resolved by having the current FSE physical move and/or tilt his or her head to match the vantage of the video. Such movement is also advantageous as it places the FSE performing the current servicing of the current medical imaging device in the same physical position respective to the current medical imaging device that was taken by the FSE in the pre-recorded video when performing the action.

In some variant embodiments, rather than the database storing historical service calls of actual servicing of customer-installed medical imaging device, it may store historical service calls which are simulated service calls, for example performed not to provide actual servicing but rather to generate the AR content. This can have the advantage of being an "ideal" service call performed by an expert service technician in a well-controlled environment. In another variant, both historical service calls and simulations may be stored.

In other embodiments, the videos may be segmented into chapters similarly to chaptering of some YouTube® videos, to enable the current FSE to jump to relevant parts of the video. Typical video playback controls such as rewind and pause are suitably provided, and optionally also zoom capability.

Other disclosed aspects relate to selection of the pre-recorded video to be presented to assist in a particular current service call. In one variant, a third party such as a remote service engineer (RSE) who sends out the current FSE may preselect the video for the current FSE. In another variant, an automatic video selector selects the video for assisting the current service call based on, for example, comparing content of a work order for the current service call with the metadata associated with the pre-recorded videos of the database. If the automatic video selector identifies more than one close video, the system may provide a user interface via which the current FSE can select the video to play back, e.g. using tactile controls on the AR glasses or headset or using an in-AR menu, for example driven by the FSE focusing on a displayed menu option and detecting this using eye trackers built into the AR glasses or headset.

In yet another embodiment, the AR can provide other information such as a summary of the current case.

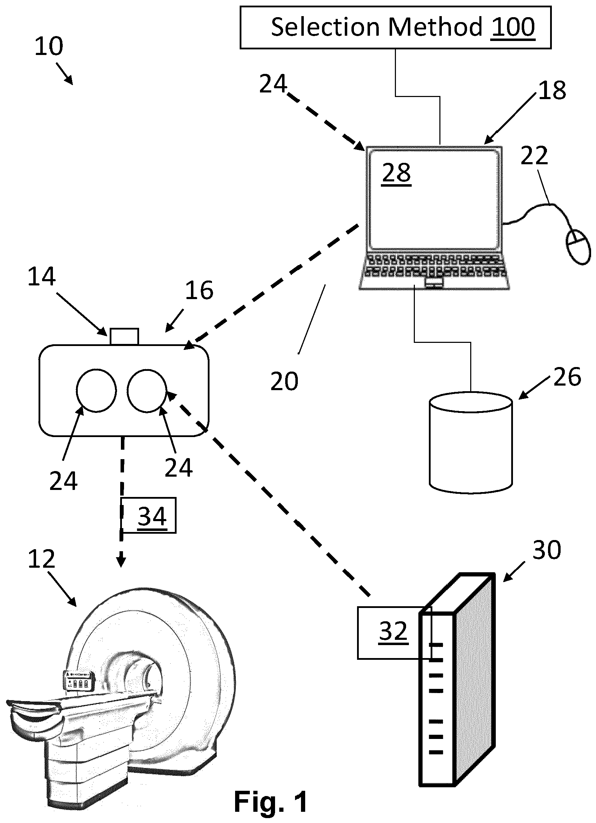

With reference to Fig. 1, an illustrative apparatus or system 10 for providing maintenance videos for one or more medical devices 12 (e.g., an MRI device or other imaging modality device) in a hospital or other medical facility is shown. To do so, the apparatus 10 includes a stereo camera 14 configured to acquire stereo images or videos of the medical device 12 and/or locations of structures within a medical facility. The stereo camera 14 typically includes multiple lenses or lens assemblies with a separate sensor for each lens that forms an image on a digital detector array (e.g. a CCD imaging array, a CMOS imaging array, et cetera) to capture 3D images. The stereo camera 14 preferably (although not necessarily) has color video capability, e.g. by having an imaging array with pixels sensitive to red, green, and blue light (or another set of colors substantially spanning the visible spectrum, e.g. 400-700 nm). The stereo camera 14 optionally may include other typical features, such as depth detection, a built-in flash (not shown) and/or an ambient light sensor (not shown) for setting aperture, ISO, and/or exposure times. FIGURE 1 also shows an electronic processing device 18, such as a workstation computer, a smartphone, a tablet, or so forth. Additionally or alternatively, the electronic processing device 18 can be embodied as a server computer or a plurality of server computers, e.g. interconnected to form a server cluster, cloud computing resource, or so forth. In other embodiments, the electronic processing device 18 can be integrated into an augmented reality heads-up (AR-HUD) device 16. Various combinations or distributions of the processing hardware are also contemplated.

The electronic processing device 18 includes typical components, such as an electronic processor 20 (e.g., a microprocessor), at least one user input device (e.g., a mouse, a keyboard, a trackball, and/or the like) 22, and at least one display device 24 (e.g. an LCD display, an OLED display, a touch-sensitive display, plasma display, cathode ray tube display, and/or so forth). In some embodiments, the display device **24** can be a separate component from the electronic processing device **18.** The display device **24** may also comprise two or more display devices.

The electronic processor **20** is operatively connected with a one or more non-transitory storage media **26.** The non-transitory storage media **26** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the electronic processing device **18,** various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a graphical user interface (GUI) **28** for display on the display device **24.**

In some embodiments, the stereo camera **14** comprises the AR-HUD device **16** with one or more AR-HUD displays comprising the display device (s) **24.** The illustrative design employs left-eye and right-eye displays **24,** but alternatively the display can be a single large window that spans both eyes. In some examples, the stereo camera **14** is mounted to the AR-HUD device **16** to provide a "first person view" so as to align the AR content with the actual view seen through the transparent display(s) **24** of the AR-HUD. In some examples, the AR-HUD device **16** can be configured as a helmet, a headband, glasses, goggles, or other suitable embodiment in order to be worn on the head of the user. The stereo camera **14** is mounted to the AR-HUD device **16** (e.g., to overlay the user's forehead, or including two stereo cameras disposed on lenses of the glasses).

Fig. 1 also shows the AR-HUD device **16** in communication with a server computer **30** comprising a non-transitory computer readable medium. The server computer **30** can comprise a database that stores a plurality of recorded historical service call videos **32** for one or more medical imaging devices (i.e., including the medical device **12).** As used herein, the term "recorded historical service call" (and variants thereof) can refer to a recorded service call performed on a "model" medical imaging device for the purpose of providing assistance to the user of the AR-HUD device **16.** The historical service call videos **32** can be annotated with information about the service calls. The historical service call videos **32** can also be pre-recorded videos (i.e., before the service calls are generated). In some embodiments, the recorded historical service call videos **32** include recorded videos of historical service calls which are simulated service calls depicting an "ideal" service call.

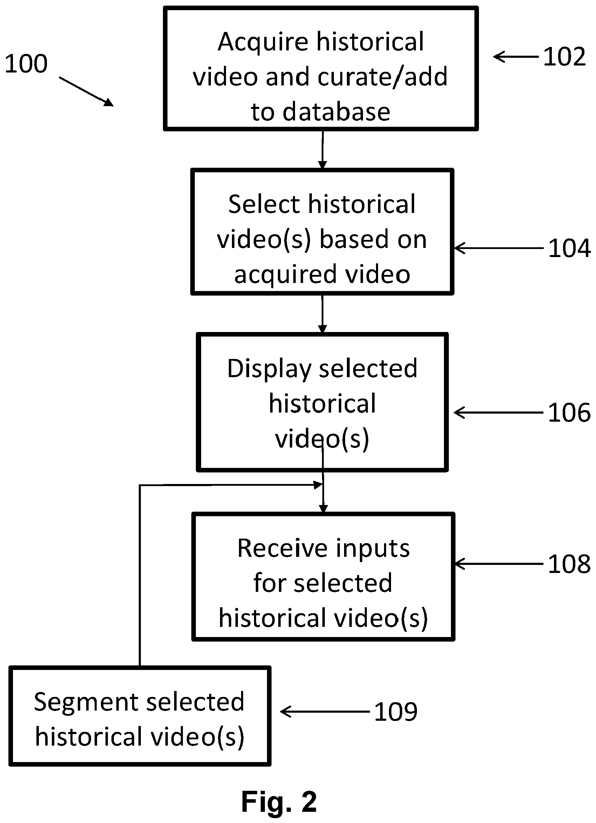

Furthermore, as disclosed herein, the non-transitory storage media **26** (or the server computer **30**) performs a method or process **100** for providing maintenance videos for one or more medical devices **12.** With reference to FIGURE 2, and with continuing reference to FIGURE 1, the server computer **30** is configured as described above to perform the method **100** for providing maintenance videos for one or more medical devices **12.** The server computer **30** stores instructions which are readable and executable by the server computer **30** to perform disclosed operations including performing the method or process **100.** In some examples, the method **100** may be performed at least in part by cloud processing.

In some embodiments, the apparatus **10** can be implemented using the AR-HUD device **16** to acquire images of the medical device **12,** and the electronic processing device **18** can be embodied as a workstation computer (or tablet) which processes the acquired images to determine the maintenance videos. In other embodiments, the electronic processing device **18** can be implemented in the AR-HUD device **16,** in which case the at least one user input device **22** might for example comprise gaze trackers (not shown) built into the AR-HUD device **16** in combination with selection buttons, menu, or other user interfacing dialogs presented as AR content and selectable by the user directing his or her gaze on the button, menu option, or so forth and staring at it for a predetermined selection time.

At an operation **102,** the camera **12** is controlled (e.g., by the server computer **30**) to acquire a video **34** of a current medical device (e.g., the medical device **12**) being serviced in a current service call, and the historical video is curated and added to the database of historical videos on the server computer **30.** For example, a video is recorded by an experienced FSE while performing a servicing task for which that FSE is particularly well-qualified. The resulting recorded video may be reviewed by the FSE or by another qualified individual (e.g., a servicing department manager) to determine whether it is of sufficiently high quality for inclusion in the server computer **30.** If so, then suitable video editing may be performed, such as extracting the video segment that recorded the service action from the overall video (which may include events recorded before and/or after the actual service action), optionally adding chapter or segment points to enable a user to jump to a particular part of the video, adding textual metadata describing the action recorded, the make/model of the imaging device whose servicing is recorded (and possibly also an indication of other similar makes/models for which the video may be applicable), date information, and so forth. These actions may in general be performed using any suitable video editing software. The resulting pre-recorded video is converted to AR content by, for example, assigning the left and right channels of the stereo video to respective left and right AR channels, reformatting the video to an AR format used by the AR-HUD device **16,** optionally compressing the video to reduce its file size and thus bandwidth used in transmitting the video, and/or so forth. It will be appreciated that the operation **102** is suitably applied for a wide range of videos acquired during the normal course of servicing of medical imaging devices of a fleet of medical imaging devices maintained by the service provider, so that over time the database accumulates a substantial amount of AR content covering a wide range of servicing actions on a range of makes/models of devices. Advantageously, the amount of additional effort beyond the service calls which are already being performed in the normal course of servicing operations is limited to the selection of the "best" videos and curating of these best videos by adding textual description, chapters, or other metadata and running automated video format conversion to AR content.

At an operation **104,** one (or more) of the historical service call videos **32** is selected based on the acquired video **34.** In one embodiment, the recorded historical service call videos **32** include metadata related to a type of medical device and a type of service performed, which were added during the curation of operation **102.** The selection operation **104** includes analyzing the metadata of the recorded historical service call videos **32,** and the historical service call video(s) **32** are selected based on having metadata most similar to metadata of the acquired video **34.**

At an operation **106,** the selected historical service call video(s) **32** are provided on the display device **24.** In one example, the selected historical service call video(s) **32** are provided on the display device **24** of the electronic processing device **18.** In another example, the selected historical service call video(s) **32** are provided on the display device(s) **24** of the AR-HUD device **16** as augmented reality content. The selected historical service call video(s) **32** can be displayed as a transparent or semi-transparent overlay on the display device **24.** Optionally, the transparency of the augmented reality content may be adjustable, for example by adjusting an alpha channel transparency value if using alpha transparency. In some examples, a summary of the current service call can be provided on the display device **24.**

At an operation **108,** one or more inputs are received by the AR-HUD device **16.** In one embodiment, a RSE wearing the AR-HUD device **16** can provide an input (i.e., via an AR user interface with which the user interacts via gaze selection) comprising a selection of one or more of the historical service call videos **32.** Alternatively, the input can be provided by the electronic processing device **18** via the at least one user input device **22** (e.g., a mouse click, a keystroke, and so forth). In another example, multiple historical service call videos **32** are displayed on the display device **24,** and the user can provide an input indicative of a selection of one of the historical service call videos **32.**

At an optional operation **109,** the selected historical service call video(s) **32** can be segmented into one or more subsections, and the received input can be indicative of fast-forwarding or rewinding to a different subsection of the selected historical service call video **32.**

In some embodiments, the AR-HUD device **16** can detect whether a gaze of a user of the AR-HUD device **16** matches a vantage point of the displayed historical service call video **32.** An instruction can then be provided on the display device **24** for the user to change the gaze to match the vantage point of displayed historical service call video **32.**

In Fig. 2, a linear workflow is shown in which the historical video acquisition and curation operation **102** precedes the selection and playback operations **104, 106, 108, 109.** However, while the selection operation **104** can only select from amongst pre-recorded videos generated by the operation **102,** it will be appreciated that the operation **102** can be considered to be ongoing, as each performed service call can itself be the source of additional video content for inclusion in the database by way of a corresponding instance of the operation **102.** Hence, the database of AR content continues to grow organically over time as the medical imaging device vendor (or other service provider) provides servicing of a fleet of medical imaging devices.

EXAMPLE

The following describes the system **10** and the method **100** in more detail. The system **10** includes a wearable displaying device comprising the AR-HUD device **16** that can be used by the FSE while performing on-site maintenance service of a physical system (e.g., a malfunctioning medical device **12).** The camera **14** of the AR-HUD device **16** acquire first-person videos of on-site maintenance operations. The AR-HUD device **16** display the pre-recorded videos **32,** with an adjustable level of transparency, in order to allow the FSE to overlap the current video **34** with the real scene, matching device areas and statuses of the medical device **12** in the video **34** and in the real environment. The videos 32 can be recordings of actual maintenance actions shot while handling real cases, or they can be recordings of simulations of all the known repair actions.

Given a case being handled, the relevant video **32** to be displayed is obtained through a retrieve mechanism implemented by the server computer **30.** This can either work on the set of historical cases, retrieving the most similar cases to the current one, and selecting the associated video **32.** Alternatively, the mechanism may use a set of metadata information associated with each simulation video **32,** and perform the matching based on these. The number of retrieved relevant video **32** may vary, from one to many.

A diagnostic session for a current service case c can include, for example, a customer input (i.e., the initial customer enquiry regarding a system malfunction), remote diagnostic findings (i.e., the outcome of a remote diagnostic action, performed by an RSE), and/or medical device **12** information (i.e., any information about the status and history of the medical device **12** being repaired, automatically produced by the system itself, e.g., log files), and so forth.

The server computer **30** stores the pre-recorded videos **32,** and is also configured to implement a function f to retrieve the most relevant video(s) **32** given the current case information. This would match the current case c with historical cases, producing a list of N relevant related cases. The videos **32** corresponding to the selected cases would then be sent to the AR-HUD device **16.**

The server computer **30** stores the actual video files, plus a mapping function between the videos and all the related case details. Historical case details can be stored in any information management system with query capabilities over the different attributes of a case, e.g., a database. The mapping can be implemented through any association mechanism, including, but not limited to, the same database where video file paths and case details are aligned in the same table or collection, or in different tables sharing a common index, e.g., using the case unique identifier as key.

The AR-HUD device **16** can be any wearable device incorporating some lenses (as a single piece, or in multiple parts), placed in from of the FSE eyes when worn, e.g., glasses. The display device **24** comprises lenses that display the video, so that the FSE can watch it through them. A camera **14** attached or incorporated in the device frame is used to record the video **34** of a repair action while executing it. The camera **14** can be a separated device from the lenses **24,** bound to it by some means. The information displayed by the lenses **24** is controlled by an interface I, including, but not limited to, keys and knobs, to adjust the video streaming and transparency, and to display, navigate and interact with other source of information available. The interface can be integrated in the lenses **24,** or embodied in a separate device, connected through the lenses, e.g., a tablet connected to the lenses via a wireless communication system.

The repository of historical cases H is a collection of historical cases $h_1, ..., h_n$ where each $h_i$ incorporates several case details, including, but not limited to (1) the customer enquiry about the manifested malfunction, (2) a RSE report of the diagnostic findings and actions performed so far, (3) information generated by the medical device **12** (i.e., log data), (4) a type of the medical device **12** and detailed hardware configurations, (5) type of installation (e.g., right/left panels, cabinet order, ceiling rails or not, etc.) - this can be either textual descriptions, or a set of images visually describing the system being handled, (6) a handedness of the engineer who performed the on-site intervention, and so forth. The current case being handled c, as processed by the RSE prior to the FSE intervention, may contain a subset of or all the details specified in historical cases. Given these prerequisites, the video retrieving function is defined in Equation 1:

$$f(c, H) = S \qquad (1)$$

where $S \subseteq H$ is an ordered set, where historical cases are ranked according to a similarity relevance score with c, computed by f itself. The size of S can be zero or more, depending on design choices. Videos **32** corresponding to the cases in S are retrieved thanks to the mapping previously described. f can be implemented as a combination of one or more information retrieval approaches, textual and/or visual, which makes use of one or more case information to evaluate similarity between c and any $h_i$.

The selected videos **32** are then prepared to be streamed on the lenses **24,** with the FSE selecting which one to play using the device interface I.

Advantageously, an FSE would be able to consult videos **32** of similar or identical repair actions to the one being performed. In addition, thanks to the ghost-mode, such videos, being first-person shot, can be overlayed with the FSE field of view, making easier for the FSE identifying sensitive areas, following, and mimicking specific maintenance procedures, and so on. This support will, in the end, make the FSE operations more accurate and faster.

In some embodiments, instead of recordings of real cases, the videos **32** can be live recorded simulations of repair actions of the known malfunctions. Instead of recording every repair action performed by an FSE, it is possible to have expert FSE performing real simulation of repair actions for all the known malfunction requiring on-site maintenance. For this embodiment, the AR-HUD device **16** would not require a camera **14** to be always mounted on the lenses frame. However, video of simulated repair action would be still first-person shot with a wearable camera.

In order to retrieve relevant videos for the current case, each simulation video **32** can be associated with all the historical cases for which the described repair action was selected. The retrieve function f would work as described above, with the exception that the case-video association is many-to-one, i.e., several cases will point to the same video.

Alternatively, each video **32** could be associated with a set of meta-information, e.g., short description of video content, system type, type of malfunction solved, and so on (instead of with a direct case information), and the matching function *f* would use such meta-information to match the current case with relevant videos.

The main technical effect of this embodiment would be to drastically reduce the number of videos stored, as well as the number of videos to search among. Potentially, there would be only one video **32** for each known repair action, so that the set of alternatives would be less noisy in terms of choices. Moreover, videos **32** would be more streamlined in what they represent, as they would show only the needed actions, without any other possible out-of-track intervention by the FSE, e.g., doing something wrong.

In some embodiments, both real-case videos and simulation videos can be implemented. To do so, two different video repositories could be used. One containing videos as recordings of actual repair actions performed by the FSE, and one containing simulation videos. In this case, each historical case $h_i$ could be paired with both actual and simulation videos. The video retrieve function *f* would work identically as for previous embodiments. Alternatively, if using meta-information for simulation videos, two separate retrieve functions can be implemented, one for actual repair action videos, and one for simulations.

This embodiment would prevent those cases where something went wrong while recording a real repair action, e.g., no video was recorded for some reasons, wrong steps were taken during maintenance, and so on. In these cases, the simulation video would serve as a backup.

In some embodiments, the historical videos **32** can be segmented in time, and each segment could be tagged with information about the part being handled to allow for retrieval of shorter videos. The repair action videos **32** can be segmented in time, so that specific segments can be retrieved independently, showing sub-actions in an entire maintenance video. In this case, each sub-video would be still paired with the related historical case and corresponding information, but a number of additional information would be associated with it as well, including, but not limited to, the unique identifier of the part being manipulated/replaced (e.g., part12NC), the type of part, the performed action, and so on. The function *f* would also exploit such information to evaluate similarity with the current case.

In this embodiment, it would be possible to retrieve only segments of video, instead of entire videos. This would help in having a more refined selection of actions. For instance, let us assume that the case being handled would require actions $a_1$, $a_2$, $a_3$ to be executed. Let us assume that these three actions are not comprised in a single video together, but they are present in three independent videos. Without this embodiment, three entire maintenance videos would be retrieved and sent to the FSE. In turn, the FSE would have to browse through the three videos, looking for the single actions. Instead, with this embodiment, the FSE would still receive three videos, but these would be shorter and already tagged with the type of action and part details, making them easy to recognize.

In some embodiments, the selection of which video(s) **32** to display can be performed by the RSE. The selection of the video(s) **32** to be played on the FSE lenses **24** can be performed by the RSE before the case is actually sent to the FSE. The RSE would select the most relevant video(s) **32** for the case being handled, either manually, e.g., the RSE chooses bases on the remote diagnostic findings and her/his experience, or semi-automatically, e.g., the retrieve function *f* is applied to generate the list of videos *S*, which is in turn additionally filtered by the RSE, based on her/his findings.

In addition, the RSE may also operate the selection from a remote command-control centre, where the RSE can check the live feed from the FSE camera **16**, i.e., the FSE point of view, overlayed with the selected video. In such a way, the RSE can actively support the FSE, as an addition to the visual references from the video.

Advantageously, the selection of relevant videos **32** incorporates also the RSE's experience. This may lead to shorter and more accurate selection of videos, as it would not rely only on automatic process. Moreover, additional live support from the RSE to the FSE is provided, possibly making the on-site maintenance operations more accurate.

In some embodiments, each historical video **32** can have a number of additional informational layers on top (extra information added manually or automatically), e.g., overlaying markers of sensitive parts or components of interest of the medical imaging device in the video, or reference points on the medical device **12**. The repair action videos **32** can be augmented with several additional information annotated over them, including, but not limited to, visual markings of sensitive parts, descriptions of action being executed, other important visual landmarks about the device and/or actions. Such tagging can be either introduced manually, e.g., through a maintenance video tagging process performed by the FSE themselves or by other experts, or automatically, by the mean of dedicated Image Processing and Computer Vision models. Hybrid approaches to this tagging are also possible, e.g., videos **32** are first automatically annotated, and manual refinement is executed in a second stage.

The additional information annotated over the videos **32** would provide extra support to the FSE. Important parts or procedures would be highlighted, making the FSE intervention sharper and faster.

In some embodiments, in addition to displaying videos **32**, the lenses **24** would also allow for overlaying current case salient information in order for the FSE to access them quickly. Possibly, also related historical case information may be displayed and navigated, using the interface *I* on the lenses **24**. Details about the historical case related to the video **32** being displayed could be reported too. Whether to display such information, and the amount of it, would be controlled by the FSE through the same interface *I* used to control the video stream. The FSE would be able to consult case details more quickly, finding salient references to the seen scene, the action to perform and the physical device.

The system **10** can be implemented for on-site assistance of any medical imaging systems of any modality (interventional X-ray (iXR), digital x-ray (DXR), magnetic resonance (MR), computed tomography (CT), advanced medical imaging (AMI), and Ultrasound). Nevertheless, it can be applied to any device for which on-site maintenance is needed and possible.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

**Claims**

1. A non-transitory computer readable medium (**30**) storing:

    data including recorded historical service call videos (**32**) for one or more medical imaging devices (**12**), the videos being annotated with information about the service calls; and
    instructions readable and executable by at least one electronic processor (**20**) to:

        control a camera (**14**) to acquire video (**34**) of a current medical device being serviced in a current service call;
        select one of the historical service call videos based on the acquired video; and
        provide, on a display device (**24**), the selected historical service call video.

2. The non-transitory computer readable medium (**30**) of claim 1, wherein the information about the service calls comprises at least one of: a visual marker or reference point indicative of a component of the medical imaging device represented in the video, a description of one or more servicing actions being executed on the medical imaging device represented in the video, a make and/or model of the imaging device whose servicing is recorded, a date of recording, a landmark on the medical imaging device represented in the video, and/or one or more segment points segmenting the video into chapters.

3. The non-transitory computer readable medium (**30**) of any one of claims 1-2, wherein the display device (**24**) comprises an augmented-reality heads-up display (AR-HUD) device (**16**) and the selected historical service call video is provided on the display device of the AR-HUD device as AR content.

4. The non-transitory computer readable medium (**30**) of any one of claims 1-3, wherein the recorded historical service call videos (**32**) include metadata related to a type of medical device and a type of service performed, and select one or more of the historical service call videos based on the acquired video (**34**) includes:

    analyzing the metadata of the recorded historical service call videos; and
    selecting one or more recorded historical service call videos having metadata most similar to metadata of the acquired video.

5. The non-transitory computer readable medium (**30**) of any one of claims 1-4, wherein select one or more of the historical service call videos (**32**) based on the acquired video (**34**) includes:
    receiving, from a remote service engineer (RSE), an input comprising a selection of one or more of the historical service call videos.

6. The non-transitory computer readable medium (**30**) of any one of claims 1-5, wherein select one or more of the historical service call videos (**32**) based on the acquired video (**34**) includes:

    selecting a plurality of historical service call videos;
    providing, on the display device (**24**), a user interface (UI) (**28**) showing the selected historical service call videos, and
    receiving an input indicative of a selection of one of the historical service call videos shown on the UI.

7. The non-transitory computer readable medium (**30**) of any one of claims 1-6, wherein the instructions further include:

    segmenting the selected historical service call video (**32**) into one or more subsections; and
    receiving an input indicative of fast-forwarding or rewinding to a different subsection of the selected historical service call video.

8. The non-transitory computer readable medium (**30**) of any one of claims 1-7, wherein the recorded historical service call videos (**32**) includes recorded videos of historical service calls which are simulated service calls depicting an ideal service call.

9. The non-transitory computer readable medium (**30**) of any one of claims 1-8, wherein the instructions further include:
providing, on the display device (**24**), a summary of the current service call.

10. An augmented-reality heads-up display (AR-HUD) device (**16**), comprising:

a camera (**14**);
the display device (**24**); and
the non-transitory computer readable medium (**30**) of any one of claims 1-8;
wherein the selected historical service call video (**32**) is provided on the display device of the AR-HUD device as augmented reality content comprising a transparent or semi-transparent overlay.

11. The AR-HUD device (**16**) of claim 10, wherein the instructions further include:

detecting whether a gaze of a user of the AR-HUD device (**16**) matches a vantage point of the displayed historical service call video (**32**); and
providing an instruction on the display device for the user to change the gaze to match the vantage point of displayed historical service call video.

12. A system (**10**) for providing maintenance videos for one or more medical devices (**12**), the system comprising:

an augmented-reality heads-up display (AR-HUD) device (**16**) including a camera (**14**) and at least one display device (**24**); and
a server computer (**30**) configured to:

control the camera to acquire video (**34**) of a current medical device being serviced in a current service call;
select one or more historical service call videos (**32**) based on the acquired video, the historical service call videos comprising pre-recorded historical service call videos for one or more medical imaging devices, the videos being annotated with information about the service calls; and
provide, on the display device, the one or more selected historical service call video as a transparent or semi-transparent overlay.

13. The system (**10**) of claim 12, wherein the recorded historical service call videos (**32**) include metadata related to a type of medical device and a type of service performed, and the server computer (**30**) is configured to:

analyze the metadata of the recorded historical service call videos; and
select one or more recorded historical service call videos having metadata most similar to metadata of the acquired video.

14. The system (**10**) of either one of claims 12 and 13, wherein the server computer (**30**) is configured to:
receive, from a remote service engineer (RSE), an input comprising a selection of one or more of the historical service call videos (**32**).

15. The system (**10**) of any one of claims 12-14, wherein the server computer (**30**) is configured to:

select a plurality of historical service call videos;
provide, on the display device (**24**), a user interface (UI) (**28**) showing the selected historical service call videos, and
receive an input indicative of a selection of one of the historical service call videos shown on the UI.

Selection Method <u>100</u>

10

24

18

22

<u>28</u>

14

16

20

<u>24</u>

26

<u>24</u>

<u>34</u>

12

30

<u>32</u>

**Fig. 1**

100

Acquire historical
video and curate/add
to database ← 102

Select historical
video(s) based on
acquired video ← 104

Display selected
historical
video(s) ← 106

Receive inputs
for selected
historical video(s) ← 108

Segment selected
historical video(s) ← 109

**Fig. 2**

Europäisches
Patentamt

European
Patent Office

Office européen
des brevets

# EUROPEAN SEARCH REPORT

Application Number

EP 22 16 7776

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/120180 A1 (KONINKLIJKE PHILIPS NV [NL]) 18 June 2020 (2020-06-18)<br>* abstract *<br>* paragraph [0003] - paragraph [0014] *<br>* paragraph [0022] - paragraph [0026] *<br>* paragraph [0031] - paragraph [0036] *<br>* paragraph [0040] - paragraph [0043] *<br>* claims 1,2 *<br>----- | 1-15 | INV.<br>G06Q10/00<br>G16H40/40<br>G06F1/16<br>G06F3/01<br>G06F3/03 |
| A | US 2021/398054 A1 (JAGGERS ADAM [US] ET AL) 23 December 2021 (2021-12-23)<br>* abstract *<br>* paragraph [0016] - paragraph [0018] *<br>* paragraph [0038] - paragraph [0040] *<br>* claims 1,2 *<br>* paragraph [0119] - paragraph [0131] *<br>----- | 1-15 | |
| A | CN 109 005 382 A (SHENZHEN GULU AUTOMOBILE MAINTENANCE TECH CO LTD) 14 December 2018 (2018-12-14)<br>* the whole document *<br>----- | 1-15 | |

TECHNICAL FIELDS
SEARCHED (IPC)

G06F
G06Q
G16H

The present search report has been drawn up for all claims

| Place of search | Date of completion of the search | Examiner |
|---|---|---|
| Munich | 26 September 2022 | Kopp, Klaus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 7776

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2022

| Patent document cited in search report | | Publication date | Patent family member(s) | | Publication date |
|---|---|---|---|---|---|
| WO 2020120180 | A1 | 18-06-2020 | CN | 113168906 A | 23-07-2021 |
| | | | EP | 3895174 A1 | 20-10-2021 |
| | | | US | 2022020482 A1 | 20-01-2022 |
| | | | WO | 2020120180 A1 | 18-06-2020 |
| US 2021398054 | A1 | 23-12-2021 | NONE | | |
| CN 109005382 | A | 14-12-2018 | NONE | | |

For more details about this annex : see Official Journal of the European Patent Office, No. 12/82